## Europäisches Patentamt

## European Patent Office

## Office européen des brevets

(19)

(11) Veröffentlichungsnummer: **0 094 682**
**B1**

(12) # EUROPÄISCHE PATENTSCHRIFT

(45) Veröffentlichungstag der Patentschrift:
**19.11.87**

(51) Int. Cl.⁴: **A 61 M 1/00,** A 61 M 1/02

(21) Anmeldenummer: **83104875.6**

(22) Anmeldetag: **17.05.83**

(54) **Gerät zur Aufnahme und Reinfusion von Blut.**

(30) Priorität: **17.05.82 DE 3218561**

(43) Veröffentlichungstag der Anmeldung:
**23.11.83 Patentblatt 83/47**

(45) Bekanntmachung des Hinweises auf die Patenterteilung:
**19.11.87 Patentblatt 87/47**

(84) Benannte Vertragsstaaten:
**AT BE CH FR GB IT LI NL SE**

(56) Entgegenhaltungen:
**EP - A - 0 040 427**
**DE - B - 1 766 217**
**FR - A - 2 195 457**
**US - A - 2 597 715**
**US - A - 2 999 500**
**US - A - 3 774 611**
**US - A - 4 033 345**
**US - A - 4 047 526**

(73) Patentinhaber: **SOLCO BASEL AG, Rührbergstrasse 21,
CH-4127 Birsfelden (CH)**

(72) Erfinder: **Marx, Günter H., Dr.-Ing.,
G.-Caracciola-Strasse 10, D-8035 Gauting (DE)**

(74) Vertreter: **LOUIS, PÖHLAU, LOHRENTZ & SEGETH,
Ferdinand-Maria-Strasse 6, D-8130 Starnberg (DE)**

**Beschreibung**

Die Erfindung betrifft ein Gerät zur Aufnahme und Reinfusion von eigenem Blut eines Patienten, z.B. während einer Operation, mit den Merkmalen gemäss dem Oberbegriff des Patentanspruches 1 oder 7.

Bei Operationen, insbesondere Herzoperationen, aber auch bei schweren Verletzungen kommt es sehr häufig zu Blutverlusten des Patienten, die durch eine sofortige Transfusion kompensiert werden müssen, wenn das Leben des Patienten nicht gefährdet werden soll. Insbesondere bei Operationen erfolgt der Blutverlust in einer Weise, dass das verlorene Blut noch zur Verfügung steht, nämlich in Form von Blutansammlungen in Körperhöhlen des Patienten, die für den Chirurgen zugänglich sind. Es ist deshalb schon bekannt, das Blut aus derartigen Blutansammlungen wieder aufzunehmen und demselben Patienten zu reinfundieren. Ein solcher Vorgang des Aufsammelns und der erneuten Reinfusion von bzw. zu ein und demselben Patienten wird auch als intraoperative Autotransfusion bezeichnet.

Zur Durchführung der Autotransfusion ist es bekannt, insbesondere bei Herzoperationen, den Patienten an Herz-Lungenmaschinen anzuschliessen, durch welche das Blut von darin installierten Pumpen angesaugt und dem Patienten wieder zugeführt wird. Einerseits sind derartige Herz-Lungen-Maschinen jedoch ausserordentlich teuer und stehen daher nicht in jedem Operationssaal zur Verfügung, andererseits erfährt trotz schonender Behandlung in derartigen Maschinen das Blut, das eine sehr empfindliche und leicht zu schädigende Flüssigkeit ist, auf dem verhältnismässig langen zu durchlaufenden Weg doch eine gewisse Beeinträchtigung. Es ist daher bereits ein Gerät mit den Merkmalen gemäss dem Oberbegriff des Patentanspruches 1 bekannt (Prospekt 1981 «Solcotrans» der Firma SOLCO BASEL AG.), bei dem das Blut über einen Saugkopf, eine daran angeschlossene Saugleitung und einen Sauganschluss in einen formsteifen Behälter eingesaugt wird. Dieser Behälter steht über mindestens einen weiteren Anschluss mit einer Unterdruckquelle in Verbindung, durch welche der zum Ansaugen des Blutes notwendige Unterdruck im Inneren des Behälters erzeugt wird. Ist der Behälter, der beispielsweise einen Fassungsinhalt von 2000 ccm aufweist, ganz oder weitgehend gefüllt, so wird die Saugleitung abgeklemmt, die zur Unterdruckquelle führende Leitung von dem weiteren Anschluss entfernt und an diesen weiteren Anschluss ein herkömmliches Infusionsbesteck, ggf. mit einem vorgeschalteten Blutfilter, angesetzt, durch das dem Patienten in üblicher Weise das im Behälter befindliche Blut infundiert wird.

Dieses bekannte Gerät hat zwar den Vorteil, dass das Blut auf kurzem Wege aufgenommen werden kann, so dass eine Schädigung weitgehend vermieden wird, jedoch kann es aufgrund einiger Nachteile nicht voll zufriedenstellen. Ein wesentlicher Nachteil besteht darin, dass mit diesem Gerät die Reinfusion des im Behälter aufgenommenen Blutes nur auf dem Weg einer herkömmlichen Transfusion in ein Blutgefäss des Patienten möglich ist. Da eine Autotransfusion der hier besprochenen Art jedoch immer dann vorgenommen wird, wenn grössere Blutverluste des Patienten vorliegen, ist es zugleich von wesentlicher Bedeutung, dass dem Patienten das Blut auch in kürzester Zeit wieder reinfundiert wird, da andernfalls während eines erheblichen Zeitraumes ein beträchtliches und daher lebensgefährdendes Defizit an Körperblut entstehen kann. Dies ist nur mit einer Druckinfusion des aufgenommenen Blutes zu erreichen, für die aber das bekannte Gerät nicht vorgesehen ist und welche überdies damit ohne die Gefahr einer Luftembolie des Patienten nicht ausführbar ist.

Es ist weiterhin auch schon ein dem vorstehend besprochenen Gerät ähnliches Gerät für die intraoperative Autotransfusion bekannt geworden, bei dem das Blut des Patienten zunächst in einen formsteifen Behälter durch Unterdruck eingesaugt und anschliessend aus diesem Behälter mittels einer Blutpumpe dem Patienten reinfundiert wird (US-A-4 047 526 und US-A-4 033 345). In einer Ausführungsform dieses bekannten Geräts wird die an den formsteifen Behälter angeschlossene Blutpumpe durch einen axial zusammendrückbaren Faltenbalg oder dgl. gebildet, der lösbar mit dem formsteifen Behälter verbunden ist und mittels dessen ein Vakuum erzeugbar ist, durch welches das Vakuum in dem formsteifen Behälter überwunden werden kann. Dieser Faltenbalg oder dgl. wird nach seiner Füllung mit Blut von dem formsteifen Behälter gelöst und zum Zweck der Reinfusion dem Anästhesisten ausgehändigt. In einer anderen Ausführungsform dieses bekannten Geräts wird die Blutpumpe durch einen weiteren Behälter gebildet und unlösbar mit dem formsteifen Behälter verbunden. Sie weist einen innen liegenden dritten, nachgiebig verformbaren Behälter sowie an Einlass und Auslass jeweils ein Rückschlagventil auf. Der nachgiebig verformbare Behälter wird durch abwechselndes Anlegen von Überdruck und Unterdruck an einen Anschluss zusammengedrückt und wieder expandiert, so dass hierdurch unter Vermittlung der beiden Rückschlagventile das Blut aus dem erstgenannten formsteifen Behälter abgesaugt und dann durch das Auslassventil dem Patienten weitergegeben wird.

Zwar ist es mit diesem bekannten Gerät möglich, das Blut wieder schnell zuzuführen, jedoch ist nachteilig daran, dass das Blut mit einer verhältnismässig grossen blutfremden Oberfläche in Kontakt kommt, nämlich in dem formsteifen Behälter und in der Blutpumpe, und dass es überdies durch das in der Blutpumpe erzeugte und das Vakuum im formsteifen Behälter noch übersteigende Vakuum beeinträchtigt wird.

Zum Stand der Technik gehört weiterhin ein älterer Vorschlag für ein Gerät zur Durchführung der Autotransfusion, das einen formsteifen Behälter und einen darin aufgenommenen leicht verformbaren Behälter aufweist (EP-A-0 072 738). Mit dem Innenraum des verformbaren Behälters sind ein Sauganschluss für eine Saugleitung zum Ansaugen von Blut des Patienten und ein Anschluss für eine Unterdruckquelle verbunden, um einen zum Ansaugen des Blutes notwendigen Unterdruck in dem verformbaren Behälter zu erzeugen. Der äussere formsteife Behälter weist eine feststehende untere sowie eine verschiebbare obere Wandung auf. Zwischen der verschiebbaren oberen Wandung und einer am Behälter befestigbaren Kappe ist eine Druckfeder aufgespannt, und die verschiebbare Wandung ist durch einen äusseren Stellring mittels eines Bajonettverschlusses in einer bestimmten Stellung an dem Behälter verriegelbar, in der die Druckfeder gespannt ist. Der leicht verformbare innere Behälter ist an der feststehenden unteren Wandung und an der verschiebbaren oberen Wandung des formsteifen Behälters befestigt und hat in der Stellung, in der die Druckfeder gespannt ist, ein solches Längen/ Querschnitts-Verhältnis, dass er auch bei in seinem Inneren herrschendem Unterdruck nicht bis zur gegenseitigen Anlage seiner Wände und damit zur Abdichtung kollabieren kann. Soll das in den verformbaren Behälter eingesaugte Blut dem Patienten wieder reinfundiert werden, dann wird durch Drehen des Stellringes die verschiebbare obere Wandung freigegeben, so dass sie durch die Kraft der Feder nach unten geschoben wird. Hierdurch wird zunächst die in dem verformbaren Behälter enthaltene Luft nach oben ausgedrückt und anschliessend durch die weitere Entspannung der Feder das Blut dem Patienten infundiert.

Durch die beschriebene Gestaltung dieses bekannten Geräts wird zwar ein Kollabieren des inneren Behälters verhindert, jedoch wird dadurch das Behältervolumen so klein, dass bei einigermassen handlicher Grösse des Geräts nur eine verhältnismässig geringe Blutmenge aufgenommen werden kann. Auch ist es kaum möglich, die für die Druckinfusion des angesaugten Blutes gedachte Druckfeder richtig zu dimensionieren. Wird die Feder ausreichend stark vorgespannt, damit sie auch am Ende ihres Federweges noch eine ausreichende Druckkraft auf den das Blut enthaltenden verformbaren Behälter ausüben kann, dann besteht die Gefahr, dass die verschiebbare Wandung nach der Auslösung des Stellringes zu stark auf den verformbaren Behälter schlägt, so dass dieser beschädigt wird. Wählt man die Vorspannung hingegen geringer, so kann ein wesentlicher Teil des angesaugten Blutes nicht mehr unter ausreichendem Druck infundiert werden.

Schliesslich ist auch bereits ein Gerät zum Ansaugen von Körperflüssigkeiten eines Patienten bekannt, das nicht zur Reinfusion dient (FR-A-2 195 457), bei dem in einem luftdicht verschliessbaren formsteifen Behälter ein Beutel angeordnet ist, in den die Körperflüssigkeit eingesaugt werden soll. Zu diesem Zweck steht das Innere des Beutels durch den formsteifen Behälter hindurch mit einer Saugleitung in Verbindung und zur Erzeugung des zum Ansaugen notwendigen Unterdruckes ist an dem formsteifen Behälter ein Unterdruckanschluss befestigt. Um den erzeugten Unterdruck auch im Inneren des Beutels wirksam werden zu lassen, ist dieser an seiner Oberseite mit mehreren Öffnungen versehen. Infolge des dadurch bedingten Druckgleichgewichtes zwischen Innen- und Aussenseite des Beutels kann dieser sich bei zunehmendem Flüssigkeitsstand darin verformen, so dass die weitere Ansaugung von Körperflüssigkeit beeinträchtigt wird. Vor allem aber ist auf diese Weise eine Reinfusion von angesaugtem Blut nicht möglich.

Der Erfindung liegt deshalb die Aufgabe zugrunde, ein Gerät zur Durchführung der Autotransfusion zu schaffen, durch das – wie bei dem eingangs beschriebenen bekannten Gerät – das Blut auf kurzem Wege und daher ohne eine Schädigung vom Patienten aufgenommen werden, diesem jedoch auf ebenso kurzem Weg und ohne Schädigung in kurzer Zeit wieder zugeführt werden kann.

Zur Lösung dieser Aufgabe schlägt die Erfindung zwei im Prinzip gleichwirkende, jedoch konstruktiv sich voneinander unterscheidende Ausführungsformen des Geräts vor, die im kennzeichnenden Teil der Patentansprüche 1 bzw. 7 angegeben sind.

Bei der ersten Ausführungsform der Erfindung wird aufgrund des in dem formsteifen Behälter sowie innerhalb des leicht verformbaren Behälters wirkenden Unterdrucks Blut über den Sauganschluss angesaugt. Der im Behälter wirkende Unterdruck kann nicht zu einem Kollabieren des leicht verformbaren Behälters führen, da während des Ansaugevorganges der weitere Raum zwischen der Aussenseite des leicht verformbaren Behälters und der Innenwand des formsteifen Behälters nach aussen hin verschlossen ist, z.B. durch einen auf den Druckanschluss aufgesetzten Stopfen oder dadurch, dass der Druckanschluss während des Ansaugevorganges an eine Quelle tieferen Unterdrucks angeschlossen ist. Ist der Behälter mit Blut gefüllt, so wird die Saugleitung abgeklemmt oder der Sauganschluss selbst auf eine beliebige Weise, z.B. durch ein Ventil, verschlossen und die Verbindung mit der Unterdruckquelle gelöst. Ausserdem wird nunmehr an den Druckanschluss eine Druckquelle, z.B. ein manuell betätigbares Gummiball-Handgebläse angeschlossen, so dass dem weiteren Raum ausserhalb des leicht verformbaren Behälters Druckmedium in steuerbarer Menge und mit steuerbarem Druck zugeführt werden kann. Da der formsteife Behälter durch den Druck dieses Druckmediums keine Verformung erfährt, wird auf das in dem leicht verformbaren Behälter enthaltene Blut ein dem Druck des Druckmediums entsprechender Druck ausgeübt, durch den das Blut nach dem Anschluss an den Patienten über ein Druckinfusionsbesteck und ein ggf. davorgeschaltetes Blutfilter in einem gewünscht kurzen Zeitraum reinfundiert werden kann. Die Druckbeaufschlagung des in dem Behälter enthaltenen Blutes erfolgt dabei grossflächig von aussen her über den leicht verformbaren Behälter und somit ohne eine Quetschung der roten Blutkörperchen und auch ohne eine sonstige Schädigung der Blutbestandteile, die häufig beim Durchlauf durch Pumpen und durch den Kontakt mit blutunverträglichen Werkstoffen zu erwarten sind. Es versteht sich, dass der mit dem Blut in Berührung kommende innere, leicht verformbare Behälter, der vorzugsweise ein Beutel ähnlich den herkömmlichen Blutbeuteln für die Bluttransfusion ist, aus einem blutfreundlichen Werkstoff, z.B. Polyäthylen, Silicongummi, PUR, PVC, gefertigt ist.

Der den inneren Behälter bildende Beutel ist zweckmässigerweise so geformt, dass er im unverformten Zustand der Kontur der Innenwandung des formsteifen Behälters weitgehend folgt und mit der Innenwand des formsteifen Behälters – vorzugsweise in der Nähe des Sauganschlusses und des weiteren Anschlusses – dicht verbunden ist. Die Verbindung kann durch Kleben oder durch Verschweissen der entsprechend gewählten Kunststoffe für den formsteifen Behälter und den leicht verformbaren Behälter erfolgen. Eine andere, vom fertigungstechnischen Standpunkt aus vorteilhafte Weiterbildung der geschilderten ersten Ausführungsform sieht vor, dass in den beispielsweise aus einer Kunststoffolie hergestellten inneren, leicht verformbaren Beutel an beiden Stirnseiten oder Enden aus hartem Werkstoff bestehende Anschlussstutzen eingeschweisst sind, deren Aussenfläche eine Dichtfläche bildet, die mit entsprechenden Dichtflächen an Durchlässen des formsteifen ausseren Behälters zusammenwirken. Dies kann dadurch erfolgen, dass in eine der beiden Dichtflächen Dichtringe oder dgl. eingelegt sind. Es ist aber auch möglich, an dieser Stelle eine Kunststoffverschweissung oder eine Verklebung vorzusehen.

Anstelle eines Beutels kann für den leicht verformbaren inneren Behälter auch ein axial zusammendrückbarer Faltenbalg verwendet werden, an dem zumindest einer der beiden Anschlüsse mittels eines Anschlussstutzens ausgebildet ist. Wenn die Anschlüsse des Faltenbalges an den beiden Stirnseiten liegen, was nicht zwingend ist, muss in diesem Fall der eine Anschluss gleitend und abgedichtet in einem entsprechenden Durchlass des formsteifen Behälters geführt sein, damit das enthaltene Blut zum Zweck der Reinfusion durch Einleitung von Druckmedium in den weiteren Raum ausgefördert werden kann.

Die zweite grundsätzliche Ausführungsform der Erfindung geht von dem eingangs geschilderten bekannten Gerät aus, bei dem ein formsteifer Behälter mit einem Sauganschluss zum Ansaugen von Blut und einem weiteren Anschluss zur Erzeugung eines Unterdruckes in dem Behälter vorgesehen ist. Während jedoch sowohl bei dem bekannten Gerät als auch bei der vorstehend beschriebenen ersten Ausführungsform der Erfindung die Formsteifigkeit des Behälters durch eine entsprechende Wahl des Werkstoffes und der Wanddicke des Behälters erzielt wird, ist nach der weiteren grundsätzlichen Ausführungsform der Erfindung vorgesehen, dass der formsteife Behälter ein axial zusammendrückbarer Faltenbalg ist, dessen axiale Formhaltigkeit durch eine an seinen gegenüberliegenden Enden befestigbare starre Halterung bewirkt ist. Dabei besteht der Faltenbalg aus einem Werkstoff ausreichender Biegefestigkeit und seine Wandstärke ist so gewählt, dass durch eine filmscharnierartige Ausbildung an den Biegekanten der einzelnen Falten zwar die erwähnte axiale Zusammendrückbarkeit des Faltenbalges erzielbar ist, jedoch eine ins Gewicht fallende Verformung in radialer Richtung aufgrund der dadurch erzielten Steifigkeit nicht vorliegt. Durch eine starre Halterung, z.B. ein Gestell oder einen Rahmen, in welche der Faltenbalg im expandierten Zustand so eingesetzt werden kann, dass die Halterung Formschlusselemente an den Stirnseiten des Faltenbalges festhält, kann erreicht werden, dass auch bei Anlegen des Unterdruckes der Faltenbalg seine expandierte Stellung beibehält und somit Blut über den Sauganschluss in sein Inneres eingesaugt werden kann. Ist der Faltenbalg dann ganz oder weitgehend gefüllt, so erfolgt das Verschliessen in ähnlicher Weise wie das vorstehend in Zusammenhang mit der ersten Ausführungsform nach der Erfindung geschildert ist. Die Reinfusion des Blutes wird aber jetzt nicht durch Anschluss an eine Druckmediumquelle durchgeführt, sondern durch ein Zusammendrücken des Faltenbalges in axialer Richtung. Dieses Zusammendrücken kann auf verschiedenartige Weise erfolgen, z.B. manuell durch den Anästhesisten, durch Einsetzen in eine Druckvorrichtung, z.B. eine aufblasbare Druckmanschette usw. Insbesondere das manuelle Zusammendrücken des Faltenbalges hat den Vorteil, dass der Anästhesist den Druck gefühlvoller steuern kann als dies durch die Zuführung von Druckluft möglich ist.

Ein weiterer Nachteil der eingangs beschriebenen bekannten Geräte besteht darin, dass nach dem Einsaugen einer Blutansammlung in den formsteifen Behälter ein weiteres Ansaugen von Luft zwar dadurch verhindert werden kann, dass die Saugleitung mittels einer Klemme verschlossen wird, jedoch der Unterdruck von der Unterdruckquelle her nach wie vor im Inneren des formsteifen Behälters voll wirksam ist. Hierdurch besteht ebenfalls eine nicht geringe Gefahr der Schädigung des Blutes, da z.B. die roten Blutkörperchen aufgrund des Unterdruckes übermässig expandieren und sogar platzen können. Nach einer weiteren Ausgestaltung der Erfindung ist daher vorgesehen, dass bei beiden geschilderten grundsätzlichen Ausführungsformen in der zur Unterdruckquelle führenden Unterdruckleitung ein Druckbegrenzer zur Begrenzung des im formsteifen Behälter einstellbaren Unterdruckes angeordnet ist. In einer sehr einfachen Ausführungsform wird ein solcher Druckbegrenzer durch ein Stück eines bei einem vorbestimmten Unterdruck kollabierenden Schlauches aus Silikongummi gebildet. Wird ein für das Blut kritischer Unterdruckwert erreicht, so wird das Schlauchstück aus Silikongummi durch den herrschenden Aussendruck zusammengepresst und eine weitere Evakuierung des formsteifen Behälters unterbunden.

Es versteht sich, dass bei beiden vorstehend geschilderten grundsätzlichen Ausführungsformen der Erfindung die jeweiligen Behälter aus physiologisch unbedenklichen Werkstoffen, z.B. Polyäthylen, Polyurethan oder Polyamid gefertigt sind und vorzugsweise auch durchscheinend oder durchsichtig sind, damit die Standhöhe des Blutes darin überwacht werden kann.

Weitere Vorteile und Merkmale der vorliegenden Erfindung ergeben sich aus der nachfolgenden Beschreibung der bevorzugten Ausführungsbeispiele anhand der beiliegenden Zeichnungen sowie aus weiteren Unteransprüchen. Bezüglich Einzelheiten, die in der nachfolgenden Beschreibung nicht ausdrücklich erwähnt, jedoch aus den Zeichnungen ersichtlich sind, wird auf die Zeichnungen Bezug genommen.

In den Zeichnungen zeigen:

Fig. 1 einen teilweisen Längsschnitt durch die Behälteranordnung einer ersten Ausführungsform des Geräts nach der Erfindung, wobei weitere Bestandteile des Geräts der Übersichtlichkeit halber weggelassen sind, und

Fig. 2 einen schematischen Längsschnitt durch die Behälteranordnung gemäss einer zweiten Ausführungsform der Erfindung.

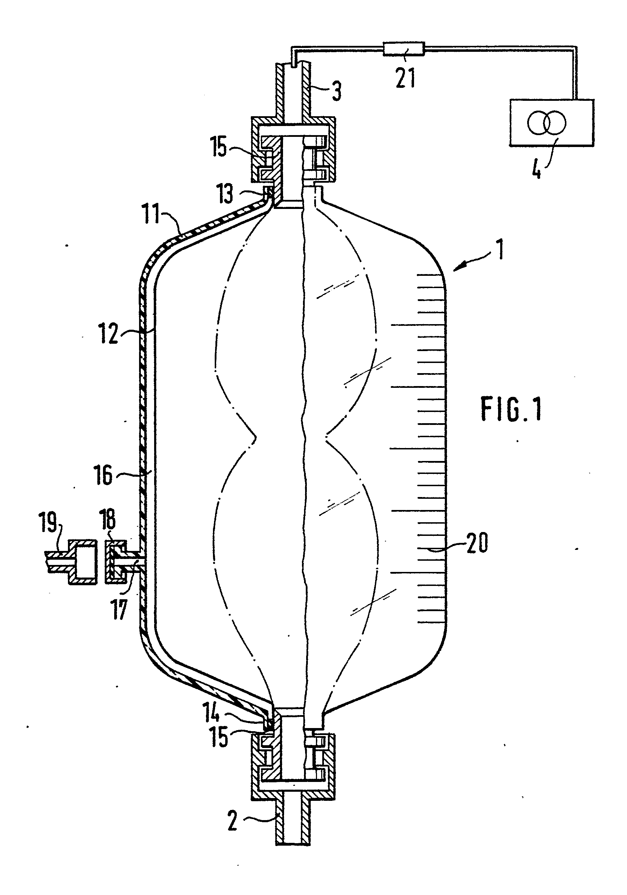

Das in Fig. 1 teilweise dargestellte Gerät zur Aufnahme und Reinfusion von Blut besteht aus einer im Ganzen mit 1 bezeichneten Behälteranordnung, an die über eine Saugleitung 2, die durch eine nicht dargestellte Klemme verschliessbar ist, ein ebenfalls nicht dargestellter Saugkopf angeschlossen ist. Weiterhin ist die Behälteranordnung 1 über eine lösbar befestigte Unterdruckleitung 3 an eine nur schematisch gezeichnete Unterdruckquelle 4, z.B. eine Vakuumpumpe, anschliessbar. Schliesslich wird das Gerät vervollständigt durch ein an sich bekanntes, ebenfalls nicht dargestelltes Infusionsbesteck, ggf. mit vorgeschaltetem Blutfilter, das anstelle der Saugleitung 2 oder der Unterdruckleitung 3 an die Behälteranordnung 1 angeschlossen werden kann und der Reinfusion von in der Behälteranordnung 1 enthaltenem Blut, wie nachfolgend geschildert wird, dient.

Die Behälteranordnung besteht aus einem äusseren formsteifen Behälter 11 und einem in dessen Innenraum angeordneten leicht verformbaren Beutel 12. Der formsteife Behälter 11, der beliebige Gestalt haben kann, zweckmässigerweise jedoch im wesentlichen zylindrisch und an den Stirnenden weitgehend verschlossen ist, weist an seinen beiden gegenüberliegenden Stirnseiten Durchlässe 13 bzw. 14 auf, die nach Art eines Durchzuges sich um ein bestimmtes Mass nach aussen erstrecken (vgl. Fig. 1). Der Beutel 12 enthält in seine gegenüberliegenden Enden eingeschweisste Anschlussstutzen 15 aus einem verhältnismässig steifen Kunststoffmaterial, an die mittels geeigneter Schlauchkupplungen die Saugleitung 2 bzw. die Unterdruckleitung 3 angeschlossen werden können. In dem in Fig. 1 ausgezogen dargestellten unverformten Zustand des Beutels 12 hat dieser eine Gestalt, die weitgehend der Innenkontur des formsteifen Behälters 11 entspricht, so dass in diesem Zustand der zwischen der Aussenseite des Beutels 12 und der Innenwandung des formsteifen Behälters 11 gebildete Raum 16 ein geringes Volumen hat. Die Innenfläche der Durchlässe 13, 14 des formsteifen Behälters 11 bildet mit der Aussenseite der Anschlussstutzen 15 eine Abdichtung des Raumes 16 nach aussen bzw. von aussen. Diese Abdichtung wird durch eine Verschweissung, eine Verklebung oder durch eingepresste Dichtungen usw. erreicht. Damit ist der weitere Raum 16 zwischen dem inneren Beutel 12 und dem formsteifen Behälter 11 lediglich über einen Druckanschluss 17 zugänglich, der in der Wandung des formsteifen Behälters 11 vorgesehen ist. Der Druckanschluss 17 ist durch einen abnehmbaren Deckel 18 verschliessbar und an ihn kann eine mit einer nicht gezeigten Druckmediumquelle verbundene Druckleitung 19 nach Abnahme des Deckels 18 angeschlossen werden.

Der äussere Behälter 11 weist eine Skala 20 auf, an der der Füllstand abgelesen werden kann.

Die Wanddicke und der Werkstoff des formsteifen Behälters 11 sind so gewählt, dass dieser den auftretenden Drücken sowohl von aussen als auch von innen her ohne eine ins Gewicht fallende Verformung standhalten kann. Hingegen besteht der Beutel 12 aus einer dünnen und leicht falt- oder verformbaren Kunststoffolie, z.B. aus Polyäthylen.

In der Unterdruckleitung 3 ist ein Schlauchstück 21 aus leicht verformbarem Silikongummi eingeschaltet, das als Unterdruckbegrenzer dient.

Die Wirkungsweise der Ausführungsform gemäss Fig. 1 ist folgende:

Zum Ansaugen von Blut aus einer Blutansammlung in einer Körperhöhle des Patienten ist die Unterdruckleitung 3 mit der Unterdruckquelle 4 verbunden, während die Saugleitung 2 mit dem nicht gezeigten Saugkopf durch den Chirurgen in die abzusaugende Blutansammlung des Patienten gehalten wird. Infolge des sich im Inneren des Beutels 12 einstellenden Unterdruckes wird dadurch das Blut in diesen Beutel eingesaugt. Da zu diesem Zeitpunkt der Raum 16 entweder durch den Deckel 18 verschlossen oder über eine nicht gezeigte Leitung ebenfalls mit der Unterdruckquelle 4 verbunden ist, kann der im Behälterinneren herrschende Unterdruck den Beutel 12 nicht einstülpen oder einfalten. Dieser behält vielmehr im wesentlichen die aus Fig. 1 ersichtliche Lage bei, da der bei einer Ablösung von der innenwandung des formsteifen Behälters 11 auftretende Unterdruck ihn im Gleichgewicht hält. Ist der Beutel 12 und damit der Behälter 11 weitgehend gefüllt oder ist die vorhandene Blutansammlung abgesaugt, so wird die Saugleitung 2 durch die nicht dargestellte Klemme verschlossen und anstelle der Unterdruckleitung 3 ein nicht gezeigtes Druckinfusionsbesteck, ggf. mit einem vorgeschalteten Blutfilter, mit dem entsprechenden Anschlussstutzen 15 verbunden. Die Nadel des Druckinfusionsbestecks ist zuvor bereits in ein Blutgefäss des Patienten eingestochen und befestigt worden. Nunmehr wird von dem Druckanschluss 17 der Deckel 18 abgenommen bzw. die Verbindung zu der Unterdruckquelle 4 gelöst und statt dessen der Anschluss der Druckleitung 19 angeschlossen. Durch gesteuerte Zufuhr von Druckmedium, z.B. Druckgas, in den Raum 16 kann jetzt Druck auf den Beutel 12 ausgeübt werden, der entsprechend auf die darin enthaltene Blutmenge wirkt und diese je nach Höhe des Druckes in kürzerer oder längerer Zeit aus dem Anschluss 13, 15 und über das Druckinfusionsbesteck dem Patienten zuführt. Der verformte Zustand des Beutels 12, der im Laufe der Zusammendrückung Falten werden wird, ist gestrichelt in Fig. 1 dargestellt.

Nach der Entleerung des Beutels 12 durch vollständiges Zusammenpressen kann der Beutel 12 durch erneutes Anlegen eines Unterdruckes an den Raum 16 über den Druckanschluss 17 wieder auf seinen ursprünglichen unverformten Zustand ausgedehnt werden.

Anstelle von Druckgas als Druckmedium kann auch eine durch eine Pumpe zugeförderte Infusionslösung das Ausdrücken des in dem Beutel 12 enthaltenen Blutes bewirken.

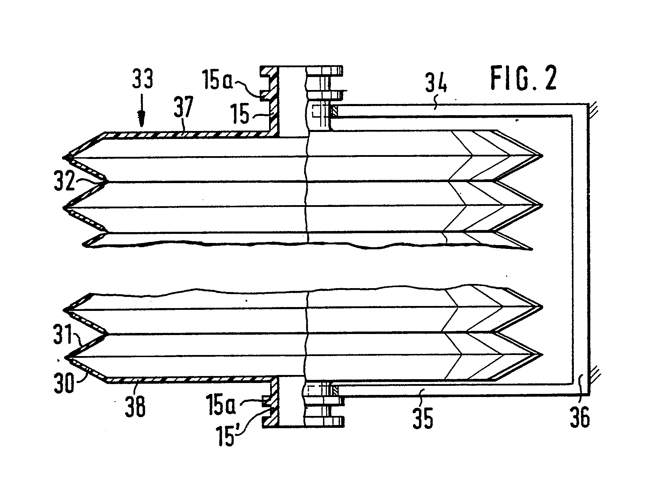

Die zweite Ausführungsform gemäss Fig. 2 weist anstelle der Behälteranordnung 1 mit den Behältern 11, 12 einen Faltenbalg 30 auf, dessen Wandstärke und Werkstoff im Bereich der Falten 31 so gewählt sind, dass diese auch bei einem im Inneren des Faltenbalges herrschenden Unterdruck nicht in radialer Richtung zusammengedrückt werden. Im Bereich der Kanten 32 hingegen, die durch die gegenseitige Verschneidung der Falten gebildet werden, ist die Wanddicke nach Art von Filmscharnieren verhältnismässig dünn gehalten, so dass der Faltenbalg 30 in axialer Richtung, d.h. in Richtung des Pfeiles 33 leicht zusammengedrückt werden kann. An seinen gegenüberliegenden Stirnseiten weist der Faltenbalg 30 Anschlüsse 15, 15' auf, die in gleicher Weise, wie das in Zusammenhang mit Fig. 1 geschildert ist, mit einer Saugleitung bzw. einer Unterdruckleitung und einem Druckinfusionsbesteck verbunden werden können. Diese Anschlüsse 15, 15' besitzen mindestens eine vorspringende Ringrippe 15a, die im expandierten Zustand des Faltenbalges 30 jeweils von dem gabelförmigen Ende eines Armes 34 bzw. 35 eines nur schematisch angedeuteten Rahmens oder Gestells 36 untergriffen werden können. Die Arme 34, 35 halten durch diese Art des Formschlusses den expandierten Zustand des Faltenbalges 30 auch dann aufrecht, wenn an den Innenraum des Faltenbalges analog zu der vorstehend geschilderten Vorgangsweise ein Unterdruck angelegt wird. Es kann daran gedacht werden, die beiden Stirnwände 37, 38 des Faltenbalges kräftiger auszubilden oder mit einer Versteifungseinlage zu versehen, um eine Einwölbung an dieser Stelle zu unterbinden.

Der Faltenbalg 30 bildet in dem aus Fig. 2 ersichtlichen Zustand das Äquivalent zu dem formsteifen Behälter 11 bei der Ausführungsform gemäss Fig. 1. Da er jedoch zugleich nach der Herausnahme aus der formschlüssigen Halterung durch die Arme 34, 35 einen leicht verformbaren Behälter entsprechend dem Beutel 12 bei der Ausführungsform gemäss Fig. 1 darstellt, erfüllt er auch dessen Funktion.

Seine Wirkungsweise ist folgende: Zunächst wird durch Anschluss einer Unterdruckquelle an einen der Anschlüsse 15, 15' ein Unterdruck im Inneren des Faltenbalges 30 erzeugt, durch wieder über die nicht gezeigte Saugleitung Blut einer Blutansammlung ins Faltenbalginnere eingesaugt wird. Ist die Blutansammlung abgesaugt oder der Faltenbalg 30 ganz oder weitgehend mit Blut gefüllt, so wird die Saugleitung abgeklemmt und anstelle der Unterdruckleitung ein Druckinfusionsbesteck in gleicher Weise angeschlossen, wie das in Zusammenhang mit Fig. 1 beschrieben ist. Nunmehr kann der Faltenbalg aus dem Gestell 36 herausgenommen werden. Die Reinfusion des darin enthaltenen Blutes zum Patienten erfolgt anschliessend durch das axiale Zusammendrükken des Faltenbalges 30 in Richtung des Pfeiles 33. Wie eingangs bereits erwähnt, kann das axiale Zusammendrücken entweder manuell oder durch Einbringen des Faltenbalges 30 in eine aufblasbare Druckmanschette oder ein sonstiges, druckausübendes Gerät erfolgen.

Die Anschlüsse 15 bzw. 15' können abweichend von den Ausführungsbeispielen auf der gleichen Stirnseite angeordnet sein, wobei dann allerdings durch eine entsprechende Verlängerung ins Innere Kurzschlüsse vermieden werden müssen. Auch ist die Erfindung keineswegs auf die in den Ausführungsbeispielen gezeigte Art von Anschlussstutzen beschränkt. Weiterhin versteht sich, dass die Eigenelastizität des Faltenbalges 30 bei der Ausführungsform gemäss Fig. 2 auch durch unmittelbar in das Kunststoffmaterial des Faltenbalges eingebettete Metallteile oder Federn bewirkt sein kann.

Alle vorstehend beschriebenen Ausführungsformen zeichnen sich durch eine sehr einfache Herstellungsart aus. Faltenbälge sind in mannigfaltigen Gestaltungsformen auf dem Markt und können z.T. nur nach geringfügigen Anpassungen (Anschlüsse) an die Erfordernisse der Erfindung angepasst werden. Bei der Ausführungsform gemäss Fig. 1 kann der äussere formsteife Behälter 11 aus zwei in einer Axialebene miteinander verbundenen Behälterhälften aufgebaut sein, wobei vor der Verbindung dieser Behälterhälften der Beutel 12 mit seinen Anschlussstutzen eingelegt wird.

Der eigentliche Zweck des hier beschriebenen Gerätes liegt in der Autotransfusion, d.h. bei der Aufnahme und Reinfusion von eigenem Blut eines Patienten, das aus Körperhöhlen oder dgl. angesaugt wird. Dadurch, dass in dem Inneren des Behälters durch die Unterdruckquelle ständig ein Unterdruck aufrecht erhalten wird, ist das Gerät auch nach der Ansaugung eines Blut/Luft-Gemisches oder bei ausschliesslicher Ansaugung von Luft infolgen fehlerhafter Haltung des Saugkopfes in der Lage, Blut anzusaugen. Jedoch ist das Gerät aufgrund seiner Konstruktion nicht ausschliesslich für die Autotransfusion verwendbar, sondern kann auch für eine Wunddränage und sonstige Dränagen und insbesondere auch für die Blutspende verwendet werden.

**Patentansprüche**

1. Gerät zur Aufnahme und Reinfusion von eigenem Blut eines Patienten, z.B. während einer Operation, mit einem einzigen formsteifen Behälter (11), der einen Sauganschluss (14, 15) mit einer daran anschliessbaren Saugleitung (2) zum Ansaugen von Blut des Patienten in den Behälter (11) sowie mindestens einen weiteren Anschluss (13, 15) aufweist, welcher mit einer Unterdruckquelle (4) verbindbar ist, um den zum Ansaugen des Blutes notwendigen Unterdruck in dem Behälter (11) zu erzeugen, dadurch gekennzeichnet, dass unmittelbar in dem einzigen formsteifen Behälter (11) ein weiterer, leicht verformbarer Behälter (12) aufgenommen ist, dessen Innenraum mit dem Sauganschluss (14, 15) und dem weiteren Anschluss (13, 15) direkt verbunden ist und der mit seiner Aussenseite und mit der Innenwandung des formsteifen Behälters (11) einen geschlossenen weiteren Raum (16) begrenzt, und dass der formsteife Behälter (11) einen mit diesem weiteren Raum (16) verbundenen und während des Ansaugens von Blut verschliessbaren Druckanschluss (17) aufweist, durch den zum Zweck der Reinfusion ein Druckmedium zuführbar ist.

2. Gerät nach Anspruch 1, dadurch gekennzeichnet, dass der weitere, leicht verformbare Behälter (12) ein Beutel ist, der im unverformten Zustand der Kontur der Innenwandung des formsteifen Behälters (11) weitgehend folgt und mit dieser Innenwandung vorzugsweise in der Nähe des Sauganschlusses (14, 15) und des weiteren Anschlusses (13, 15) dicht verbunden ist.

3. Gerät nach Anspruch 2, dadurch gekennzeichnet, dass der Beutel (12) aus hartem Werkstoff bestehende Anschlussstutzen für den Sauganschluss (14, 15) und den weiteren Anschluss (13, 15) aufweist, die in Durchlässen (13, 14) des formsteifen Behälters (11) abgedichtet eingepasst sind.

4. Gerät nach Anspruch 1, dadurch gekennzeichnet, dass der weitere, leicht verformbare Behälter ein axial zusammendrückbarer Faltenbalg ist.

Gerät nach Anspruch 4, dadurch gekennzeichnet, dass Anschlüsse an den gegenüberliegenden Stirnenden des Faltenbalges vorgesehen sind und zumindest einer der beiden Anschlüsse mittels eines Anschlussstutzens ausgebildet ist, der gleitend verschiebbar, jedoch abgedichtet in einem Durchlass des formsteifen Behälters (11) geführt ist.

6. Gerät nach Anspruch 5, dadurch gekennzeichnet, dass der gleitend geführte Anschlussstutzen an dem formsteifen Behälter (11) arretierbar ist.

7. Gerät zur Aufnahme und Reinfusion von eigenem Blut eines Patienten, z.B. während einer Operation, mit einem Behälter (30), der einen Sauganschluss (15) mit einer daran anschliessbaren Saugleitung zum Ansaugen von Blut des Patienten in den Behälter (30) sowie mindestens einen weiteren Anschluss (15') aufweist, welcher mit einer Unterdruckquelle verbindbar ist, um den zum Ansaugen des Blutes notwendigen Unterdruck in dem Behälter (30) zu erzeugen, dadurch gekennzeichnet, dass der Behälter ein radial formsteifer, axial jedoch zusammendrückbarer Faltenbalg (30) ist, dessen axiale Formhaltigkeit während des Ansaugens von Blut durch den Sauganschluss (15) durch eine starre Halterung (34, 35, 36) bewirkt ist, die in an seinen gegenüberliegenden Enden vorgesehene Vorsprünge (15a) lösbar eingreift.

8. Gerät nach einem der Ansprüche 1 bis 7, dadurch gekennzeichnet, dass in die zu der Unterdruckquelle (4) führende Unterdruckleitung (3) ein Druckbegrenzer (21) zur Begrenzung des in dem formsteifen Behälter (11, 30) einstellbaren Unterdruckes angeordnet ist.

9. Gerät nach Anspruch 8, dadurch gekennzeichnet, dass der Druckbegrenzer (21) ein Stück eines bei einem vorbestimmten Unterdruck kollabierenden Schlauches aus Silikongummi ist.

**Claims**

1. Apparatus for receiving and reinfusing a patient's own blood, for example during an operation, comprising a single container (11) which is of a rigid shape and which has a suction connection (14, 15) with a suction line (12) connectable thereto, for sucking blood from the patient into the container (11), and at least one further connection (13, 15) which can be connected to a reduced pressure source (4) in order to produce the reduced pressure in the container (11) required for sucking in the blood, characterised in that a further, easily deformable container (12) is accommodated directly in the single rigid container (11), the interior of the further container (12) being directly connected to the suction connection (14, 15) and the further connection (13, 15), the outside of the further container (12) and the inside wall of the rigid container (11) defining a further closed space (16), and that the rigid container (11) has a pressure connection (17) which is connected to the further space (16) and which can be closed during the operation of sucking in the blood and through which a pressure medium can be supplied for the purposes of the reinfusion operation.

2. Apparatus according to claim 1 characterised in that the further, easily deformable container (12) is a bag which in the undeformed condition substantially follows the contour of the inside wall of the rigid container (11) and is sealingly connected to said inside wall preferably in the vicinity of the suction connection (14, 15) and the further connection (13, 15).

3. Apparatus according to claim 2 characterised in that the bag (12) has connection members comprising a hard material, for the suction connection (14, 15) and the further connection (13, 15), said connection members being sealingly fitted into apertures (13, 14) in the rigid container (11).

4. Apparatus according to claim 1 characterised in that the further, easily deformable container is an axially compressible concertina-like member.

5. Apparatus according to claim 4 characterised in that connections are provided at oppositely disposed ends of the concertina-like member and at least one of the two connections is formed by means of a connection member which is slidably displaceably but sealingly guided in an aperture in the rigid container (11).

6. Apparatus according to claim 5 characterised in that the slidably guided connection member can be locked to the rigid container (11).

7. Apparatus for receiving and reinfusing a patient's own blood, for example during an operation, comprising a container (30) which has a suction connection (15) with a suction line connectable thereto, for sucking blood from the patient into the container (30), and at least one further connection (15') which can be connected to a reduced pressure source in order to produce the reduced pressure in the container (30), required for sucking in the blood, characterised in that the container is a concertina-like member (30) which is rigid in shape in a radial direction but which is axially compressible and whose axial stability in respect of shape, during the operation of sucking blood through the suction connection (15), is provided by a rigid holding means (34, 35, 36) which releasably engages into projections (15a) provided at its oppositely disposed ends.

8. Apparatus according to one of claims 1 to 7 characterised in that a pressure limiting means (21) for limiting the reduced pressure which can be produced in the rigid container (11, 30), is disposed in the reduced pressure line (3) leading to the reduced pressure source (4).

9. Apparatus according to claim 8 characterised in that the pressure limiting means (21) is a portion of a hose of silicone rubber, which collapses at a predetermined reduced pressure.

**Revendications**

1. Appareil de collecte et de retransfusion du sang propre d'un patient, par exemple pendant une opération, comprenant un récipient unique de forme rigide (11) qui présente un raccord d'aspiration (14, 15) avec un conduit d'aspiration (2), qui peut lui être raccordé, pour l'aspiration du sang du patient dans le récipient (11) ainsi qu'au moins un autre raccord (13, 15) qui peut être relié à une source de dépression (4) pour produire dans le récipient (11) la dépression nécessaire pour l'aspiration du sang, caractérisé en ce que, directement dans l'unique récipient de forme rigide (11) est logé un autre récipient (12) aisément déformable dont le volume interne est directement relié au raccord d'aspiration (14, 15) et à l'autre raccord (13, 15) et qui délimite par sa face externe et avec la paroi interne du récipient de forme rigide (11) un autre espace fermé (16), et en ce que le récipient de forme rigide (11) présente un raccord sous pression (17) qui est relié à cet autre espace (16) et peut être fermé pendant l'aspiration du sang et par lequel un milieu sous pression peut être alimenté en vue de la retransfusion.

2. Appareil suivant la revendication 1, caractérisé en ce que l'autre récipient (12) aisément déformable est un sac qui, à l'état non déformé, suit largement le contour de la paroi interne du récipient de forme rigide (11) et qui est relié de manière étanche à cette paroi interne, de préférence à proximité du raccord d'aspiration (14, 15) et de l'autre raccord (13, 15).

3. Appareil suivant la revendication 2, caractérisé en ce que le sac (12) présente des tubulures de raccordement en matériau dur, pour le raccord d'aspiration (14, 15) et l'autre raccord (13, 15), qui sont adaptées de manière étanche dans des passages (13, 14) du récipient de forme rigide (11).

4. Appareil suivant la revendication 1, caractérisé en ce que l'autre récipient aisément déformable est un soufflet axialement compressible.

5. Appareil suivant la revendication 4, caractérisé en ce que des raccords sont prévus sur les extrémités frontales opposées du soufflet et en ce qu'au moins un des deux raccords est réalisé au moyen d'une tubulure de raccordement qui est guidée de manière à pouvoir coulisser, de façon étanche toutefois, dans un passage du récipient de forme rigide (11).

6. Appareil suivant la revendication 5, caractérisé en ce que la tubulure de raccordement guidée de manière coulissante peut être bloquée sur le récipient de forme rigide (11).

7. Appareil de collecte et de retransfusion du sang propre d'un patient, par exemple pendant une opération, comprenant un récipient (30), qui présente un raccord d'aspiration (15) avec un conduit d'aspiration qui peut lui être raccordé pour l'aspiration du sang du patient dans le récipient (30) ainsi qu'au moins un autre raccord (15') qui peut être relié à une source de dépression, pour produire dans le récipient (30) la dépression nécessaire pour l'aspiration du sang, caractérisé en ce que le récipient est un soufflet (30) de forme rigide radialement, mais compressible axialement, dont la capacité de maintien de la forme axialement est, pendant l'aspiration du sang à travers le raccord d'aspiration (15), produite par une fixation rigide (34, 35, 36) qui entre en prise de manière détachable dans des saillies (15a) prévues à ses extrémités opposées.

8. Appareil suivant l'une des revendications 1 à 7, caractérisé en ce qu'un limitateur de pression (21) destiné à limiter la dépression ajustable dans le récipient de forme rigide (11, 30) est agencé dans le conduit sous dépression (3) qui mène à la source de dépression (4).

9. Appareil suivant la revendication 8, caractérisé en ce que le limitateur de pression (21) est un morceau d'un conduit flexible en caoutchouc de silicone qui s'aplatit à une pression réduite prédéterminée.

FIG.1

FIG. 2